# EUROPEAN PATENT SPECIFICATION

(11) **EP 1 991 122 B1**
(45) Date of publication and mention of the grant of the patent: **19.04.2017**
(21) Application number: 07752409.8
(22) Date of filing: 07.03.2007
(51) Int. Cl.: A61B 5/08, A61B 5/113, A24F 47/00

(54) **MONITORING AND QUANTIFICATION OF SMOKING BEHAVIORS**
ÜBERWACHUNG UND QUANTIFIZIERUNG VON RAUCHVERHALTEN
SURVEILLANCE ET QUANTIFICATION DES COMPORTEMENTS DE FUMEURS

(30) Priority: 09.03.2006 US 781468 P; 06.03.2007 US 682601
(43) Date of publication of application: 19.11.2008
(73) Proprietor: adidas AG, 91074 Herzogenaurach (DE)
(72) Inventor: DERCHAK, P., Alexander, Ventura, California 93001 (US)
(74) Representative: Wegner, Hans
(86) International application number: PCT/US2007/005707
(87) International publication number: WO 2007/103380

(56) References cited:
- US-A- 4 597 394
- US-A1- 2004 030 508
- US-A1- 2004 177 674
- US-A1- 2005 119 586
- EUN M LEE ET AL: "Smoking topography: Reliability and validity in dependent smokers" NICOTINE & TOBACCO RESEARCH, CARFAX, ABINGDON, GB, vol. 5, no. 5, 1 January 2003 (2003-01-01) , pages 673-679, XP009129405 ISSN: 1462-2203

## Description

### FIELD OF THE INVENTION

The present invention is related to ambulatory physiological monitoring, and in particular to automatic systems and methods that quantify a subject's overall smoking behaviors over time and that quantify characteristics of the subject's method of smoking, e.g., the nature of the subject's puffs.

### BACKGROUND OF THE INVENTION

Smoking, whether of cigarettes, cigars, pipes, or other smoking materials, is well known as a serious health problem worldwide leading to significant morbidity and mortality primarily from induced lung disease. It is advantageous to provide smokers with facilities and information to aid them to cease smoking, or to limit smoking, or to smoke in less pathogenic ways. It is also advantageous to estimate the likelihood and severity of possible lung disease in dependence on a subject's smoking behavior. It is also advantageous to monitor and to quantify the average smoking behaviors of several subjects and/or of populations of subjects for public health, research, and other purposes.

Automatic systems and methods to monitor and quantify a subject's smoking behaviors over time and to monitor and quantify the characteristics of the subject's method of smoking are not available in the prior art.

Document US 2004/0030508 A1 describes a method for measuring topographical information to provide smoking topographical information including providing a portable smoking topography measurement unit having a smoking material holder; inserting a smoking material into the smoking material holder of the portable smoking topography measurement unit; detecting each puff of the smoking material; measuring flow rate of smoking from a smoking material into a subject during each puff; computing puff information; eliminating false puffs from the puff information; computing smoking material information; and storing puff information and smoking material information in a memory. The puff information and smoking material information is transferred from the memory of the portable smoking topography measurement unit to a workstation, and the puff information and smoking material information is displayed on a display unit.

Further, US 2004/0177674 A1 relates to a cigarette monitoring device capable of monitoring individual smoking behaviour and particularly make deliveries and communicating this and other information to a remote location and/or the consumer.

Finally, US 2005/011586 A1 describes systems and methods for processing respiratory signals derived generally from respiratory plethysmography, and especially from respiratory inductive plethysmographic sensors mounted on a garment for ambulatory recording.

Objects of the present invention are to provide systems and computer-implemented methods that provide smokers with such information.

### SUMMARY OF THE INVENTION

The invention is defined in independent claims 1 and 10.

This invention includes the use of an ambulatory cardio-respiratory monitoring system to provide data that can be processed and interpreted to monitor and quantify specific characteristics of a subject's cigarette and other types of smoking. This monitoring will enable a greater understanding of the interaction between smoker and cigarette, or other smoking material. It will also aid individual smokers to cease, limit, or improve their smoking behaviors.

The invention provides methods to analyze ambulatory and/or clinical and/or laboratory monitoring data to characterize a subject's overall smoking behavior and details of the subject's smoking behavior, i.e., characteristics of the subject's individual puffs and overall puffing pattern, and how these behaviors very with different smoking materials. Overall smoking is determined in dependence on the number of puffs and on the times of their occurrence as revealed by the monitoring data. The invention identifies specific characteristics that indicate a breath is a puff, and further identifies important morphological characteristics of individual puffs. In preferred embodiments, computer-implemented methods examine tidal volume (Vt) data for puff-indicating characteristics to identify puffs. Further, it is preferred that these computer-implemented methods also examine Vt data of each puff to identify important morphological characteristics. Puff occurrence data and puff morphology data are retained and statistically summarized in order to provide characterization of all aspects of a subject's smoking behavior, i.e., a subject's overall smoking behavior and details of how the subject puffs.

Single puffs can also be monitored and quantified by determining puff inspiratory and puff expiratory volume, flow, and timing. Additionally, effects of smoking on heart rate, heart rate variability, and activity can be correlated if cardiac data is also available. Further, smoking patterns and smoker interaction with their smoking implement can provide diagnostic information related to smoking addiction and prognostic information related to likelihood of development of smoking related diseases.

Additionally, complete ambulatory and/or clinical and/or laboratory monitoring data, including cardiac, respiratory, and activity data enables detailed correlation of the activities/actions of smoker related to the inspiration, holding, and expiration of smoke and of the effect of smoking on subsequent respiratory pattern, blood oxygen saturation, heart-rate and heart rate variability, pre- and post-smoking physical activity level, and the like. Continuous monitoring during a day and night permits correlation of variations in smoking habits with respiratory pattern and subsequent sleep latency, sleep time and sleep quality.

Other aspects of the invention include monitoring the ways in which smokers smoke cigarettes of different types, such a filtered, non-filtered, menthol, low-tar, etc, and with cigars, pipes and other smoking implements. Other aspects of the invention include use of monitoring data as bio-feedback information for smokers wishing to modify their smoking behaviors in order to wean themselves from the smoking related nicotine addiction. Other aspects of the invention include the use of smoking behavior data to estimate the likelihood and severity of smoking associated diseases with more accuracy than estimates based on "packs per day" smoked. Other aspects of the invention include collecting smoking behavior data from various subject populations and storing this data in databases for various public health, research, and other purposes.

A preferred ambulatory monitoring system is capable of monitoring tidal volume, the relative contributions of rib cage and abdominal expansion to tidal volume, heart rate, ECG, motion, blood oxygen saturation, posture, and activity. Such systems are available from VivoMetrics, Inc., Ventura, CA.

### BRIEF DESCRIPTION OF THE DRAWINGS

The present invention may be understood more fully by reference to the following detailed description of the preferred embodiment of the present invention, illustrative examples of specific embodiments of the invention and the appended figures in which:
Fig. 1 illustrates a 5 minute and 45 second smoking record;
Fig. 2 illustrates morphology of a first puff;
Fig. 3 illustrates morphology of a second puff;
Fig. 4 illustrates morphology of a third puff;
Fig. 5 illustrates morphology of a fourth puff; and
Figs. 6A-B illustrates a preferred ambulatory monitoring system and exemplary embodiments of the methods of this invention.

### DETAIL DESCRIPTION OF THE PREFERRED EMBODIMENTS

Preferred embodiments of the invention are now described. These embodiments do not limit the scope of the invention, since these embodiments are illustrations of several preferred aspects of the invention. Any equivalent embodiments are intended to be within the scope of this invention. Indeed, various modifications of the invention in addition to those shown and described herein, such as alternate useful combinations of the elements described, will become apparent to those skilled in the art from the subsequent description. Such modifications and combinations are also intended to fall within the scope of the appended claims. In the following (and in the application as a whole), headings and legends are used for clarity and convenience only.

This invention is useful for subjects that are monitored while ambulatory or that are monitored while in the clinic. This invention is further useful for laboratory monitoring and studies. Although, this invention may be described as "ambulatory", or as "clinical", or as laboratory" in this application, use of these words individually in not intended to limit the applicability of this invention.

### SMOKING ANALYSIS METHODS

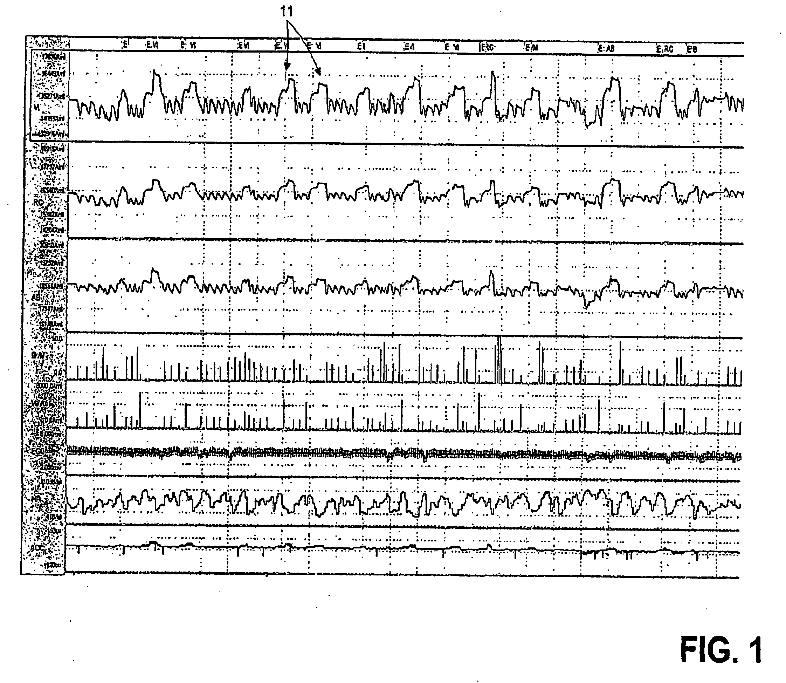

The act of smoking results in characteristic patterns of cardio-respiratory, ambulatory monitoring data. The present invention provides systems and computer-implemented methods to recognize and to quantify these characteristic patterns. These methods are now described with reference to Fig. 1-4, which illustrate exemplary smoking patterns. Preferred ambulatory monitoring devices and data analysis systems are described subsequently.

Methods of this invention can be broadly divided, first, into those that recognize puffs in cardio-respiratory data and then quantify a subject's overall smoking behavior over time (smoking behavior methods) in dependence on recognized puffs. A second group of methods are those that examine each puff in detail in order to quantify and classify the subject's manner of smoking (manner of smoking methods or puff morphology methods).

Turning first to smoking behavior methods, Fig. 1 illustrates cardio-respiratory monitoring data from a subject during a 5 min. 45 sec period of smoking. The Vt trace is the subject's tidal volume; the RC and AB traces are expansions and contractions of the subject's rib cage and abdomen, respectively; the spikes on the trace labeled Br/M mark the beginning of each breath and the height of a spike indicates the volume of the associated breath; the height of spikes in the trace labeled Ve/Vol trace indicate the expiratory volume of the associated breath; the ECG trace is the concurrent electrocardiogram; the HR trace is the heart rate; and the ACC trace is accelerometer data reflecting subject activity. During this period, the subject puffed 14 times as indicated by the highlighted bands. These puffs have distinguishing characteristics, and the computer-implemented methods of the invention recognize occurrences of puffs by examining the record for these distinguishing characteristics.

Puffs 11 illustrate the waveform characteristics and features signaling a puff. First, puffs typically have durations approximately 2.5 to 3.0 times longer than the durations of adjacent breaths, and amplitudes approximately 2 times greater than the amplitudes of adjacent breaths.

Second, puffs, have a characteristic shape, again illustrated by puffs 11. A puff is a single breath with a single inspiratory phase and a single expiratory phase. In most cases, the slope of the expiratory phase is substantially constant or slowly varying (the relative change in slope being less than approximately 50%). However, the inspiratory phase often includes two segments of similar slope separated by an intervening period of low to zero but never negative. That is, exhalation does not occur in the intervening period. However, the slope of the inspiratory phase is also often substantially constant or slowly varying. The inspiratory and expiratory phases can be separated by breath-holding periods at substantially constant but elevated lung volume.

Accordingly, one primary feature signaling a puff is a breath with a duration and amplitude both of which approximately 1.5 to 2.0 times longer or greater, respectively, than adjacent breaths. The greater these deviations, the greater is the confidence that the particular breath is a puff. A further primary signaling feature a substantially single inspiratory phase, which may be interrupted by a period at a substantially constant lung volume, and a substantially single expiratory phase. A breath holding period at a substantially constant, elevated lung volume may separate inhalation and exhalation. A puff characteristically has no further structure. In fact, further structure indicates the breath is not likely to be a puff, being instead, perhaps, a cough, or a sigh, or a period of speech, or the like. A secondary feature is heart rate which is usually decreasing over most of the puffs duration.

Thus, a puff is preferably identified as a breath having a pre-determined combination of these primary and secondary features, presence of absence of features being determined by computer-implemented examination of monitoring data. Puffs are most reliably recognized during periods of limited subject activity, which can be indicated by a motion sensor, e.g., an accelerometer, that is part of the ambulatory monitoring apparatus. In Fig. 1, the ACC trace is an accelerometer trace indicating limited subject activity. During more intense activity, normally increased minute ventilation and motion artifacts can mimic some of all of the features of puffs. Optionally, puff identification can be suspended during such activity and the period of suspension noted in the data set.

Overall smoking behaviors can be abstracted from identified puff occurrences and the times of puff occurrence. For example, the total puffs during a day can be counted. This measure is expected to be a more reliable indicator of pulmonary exposure to tobacco smoke than is the number of cigarettes (or packs) smoked per day. Further, the number smoking periods during a day as well as their length and time of occurrence can be summarized. Further, these quantitative measures of smoking can be correlated with patterns in other cardio-respiratory parameters, such as heart rate and its variability and breath rate, tidal volume, and the like and their variability. Further, for monitoring that extends over all or part of a day and the following night, quantitative measures of smoking can be correlated with objective measures of sleep time and quality in order to ascertain effects of smoking on sleep. Sleep can be assessed by time spent in various sleep stages, the number of nocturnal arousals, and the like. In preferred embodiments, sleep parameters and determined from cardio-respiratory and motion monitoring data. See <<<non-eeg sleep>>> application included in its entirety herein by reference for all purposes.

Turning next to the manner of smoking methods, puff morphology is important in improving estimates of pulmonary exposure to tobacco smoke, which are initially provided by the smoking behavior methods. Different puff morphologies are known to result in lesser or greater smoke exposure. Shorter puffs, and puffs consisting of a single inspiration followed closely by a single expiration without noticeable breath holding, limit both smoke exposure time and location of smoke exposure in the pulmonary tree. In particular, such puffs result in reduced smoke and particulate exposure of the smaller bronchioles and alveoli. These pulmonary components are more sensitive to smoke-induced injury causing ultimately to emphysema and/or COPD. On the other hand, longer puffs and puffs with breath holding provide more time for distribution and diffusion of smoke and particulates in the respiratory tree, leading to increased smoke and particulate exposure of the smaller bronchioles and alveoli receive and to increased likelihood of their injury. Further, a puff primarily due to rib cage expansion tends to lead to only limited smoke exposure in the peripheral airways. Conversely, a puff with relatively equal expansion of the rib cage and the abdomen tends to cause increase smoke exposure in the peripheral airways. Since puff morphology affects airway smoke exposure, this morphology can provide prognostic information concerning the occurrence and severity of smoking related lung diseases.

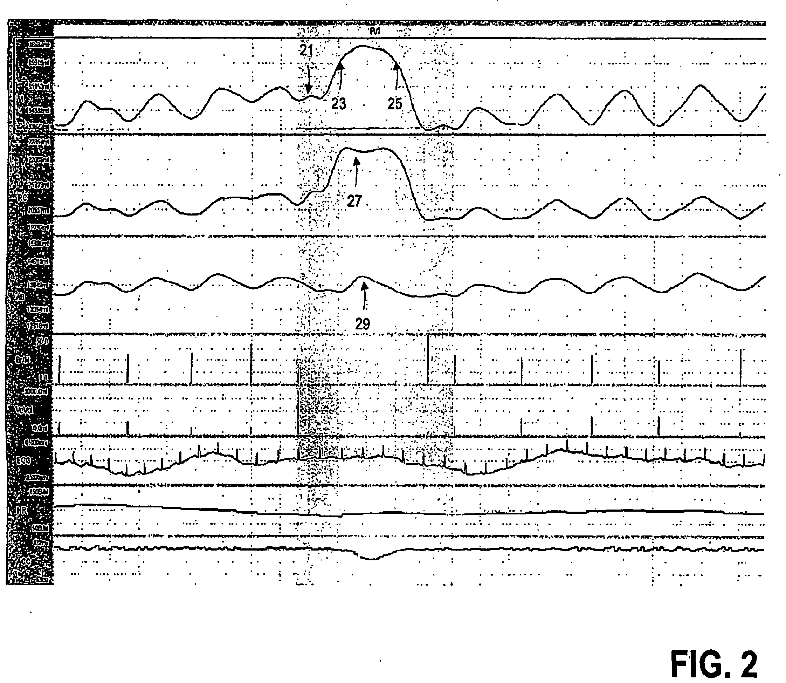

Accordingly, methods of the second class preferably recognize additional features in the tidal volume trace including, for one or more previously identified puffs: duration in seconds; presence or absence of a reduced-amplitude puff prior to the primary inhalation; relative importance of rib cage and abdominal motions in the puff; relative duration of inhalation and of exhalation; whether inhalation and exhalation are smooth or any interrupted with multiple phases; duration of breath holding if any; lung volume during breath holding; and the like. Total duration of a true puff is approximately the duration of any reduced prior puff, plus the duration of inhalation, plus the duration of any breath holding, and plus the duration of exhalation. Figs. 2-4 illustrate puffs of various types which illustrate these waveform features.

Fig. 2 illustrates a mono-phasic-type puff which is simply an inhalation followed by an exhalation without significant breath holding. This type of puff includes the following features: reduced initial puff 21, followed by primary inhalation 23 and primary exhalation 25. Both the inhalation and exhalation have slopes that are smoothly varying without evident phases or interruptions. This puff has no breath holding between inhalation and exhalation, and rib cage expansion 27 makes a significantly greater contribution to this puff than does abdominal expansion 29.

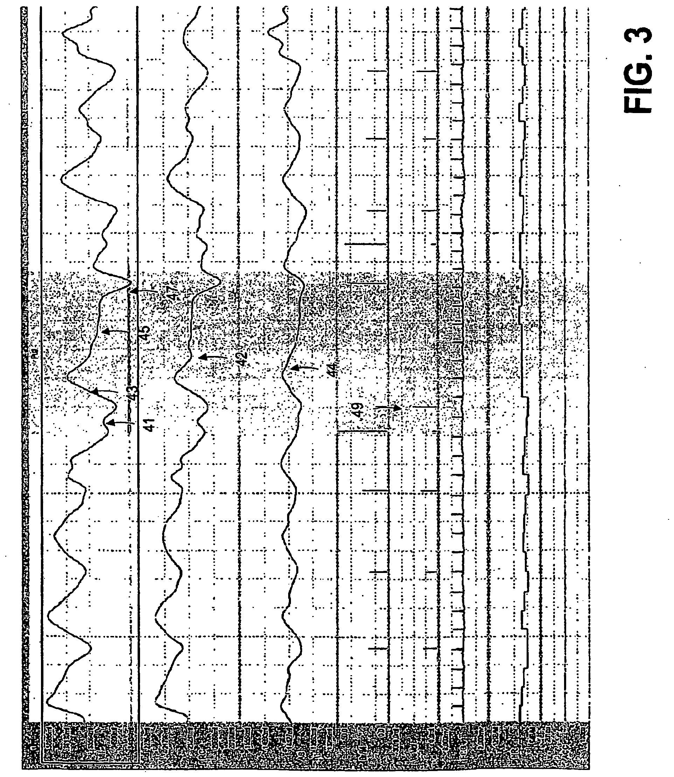

Fig. 3 illustrates a puff with a single breath holding phase but it otherwise generally similar to the puff of Fig. 2. This puff includes the following features: reduced initial puff 41; inhalation 43 with smoothly varying slope; breath holding 45 after partial expiration; and exhalation 49. Again, the inhalation and exhalation have smoothly varying slopes with the lung volume at the end of exhalation lower than the lung volume at start of inhalation. Breath-holding-period duration is less than 50% of puff duration and includes a slow exhalation; it is therefore a less prominent breath holding. The height of indicator 49 is proportional to expiratory volume and time of expiration. Here, the contribution of rib cage expansion 42 and abdominal expansion 44 are more comparable although rib cage expansion still dominates.

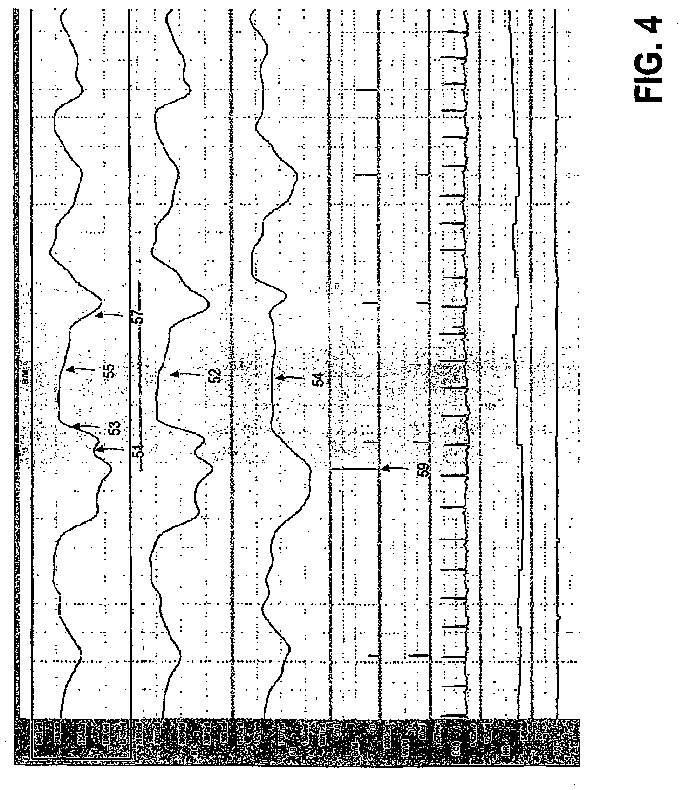

Fig. 4 illustrates a puff with more prominent breath holding. Here, breath holding 55 is more prominent: it extends for more than half the pulse duration; lung volume during breath holding is approximately at an end-inspiratory volume comparable to adjacent breaths; and the lung volume is substantially constant during breath holding. Also, the contribution of rib cage expansion 52 is here only marginally greater than the contribution of abdominal expansion 54. The height of indicator 59 represents a large expiration prior to inhalation. Inhalation 53 and exhalation 57 again have smoothly varying slopes with phases or interruptions.

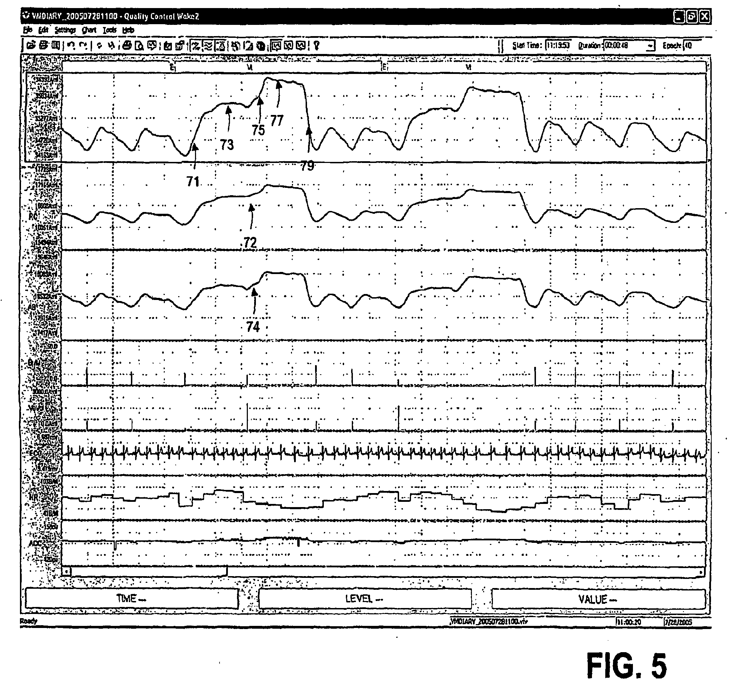

Fig. 5 illustrates very prominent breath holding at high lung volumes along with a biphasic inhalation. The inhalation displays an initial phase 71 followed by a breath holding interruption 73 at an approximately constant lung volume that is considerably higher than the end-inspiratory volume of adjacent breaths. Inhalation resumes 75 drawing more smoke into the lungs and distributing the already-present smoke further into the lung periphery. Following this inhalation phase is a further period of breath holding 77 at an even higher lung volume. These breath holding periods inhale a volume of smoke that is 2 to 3 times the tidal volume of adjacent normal breaths. This puff ends with smooth expiration 79 back to the lung volume and the beginning of inhalation. Here, approximately equal rib cage 72 and abdominal 74 expansions are needed to inhale and hold this significant volume of smoke.

Accordingly, a basic morphological classification of puffs preferably includes at least the duration and volume of the puff in comparison to adjacent normal breaths, and the relative duration of breath holding and the lung volume during breath holding, again in comparison to adjacent normal breaths.

### PREFERRED SYSTEMS

Preferred ambulatory monitoring and data analysis systems are now described which gather the data analyzed by the described smoking behavior and puff morphology methods. Physiological data can be gathered by a wide variety of monitoring systems designed for, e.g., in-hospital use, or in-clinic use, or ambulatory use, or other uses. Without limitation or prejudice, however, the following description is largely in terms of preferred monitoring systems for ambulatory use.

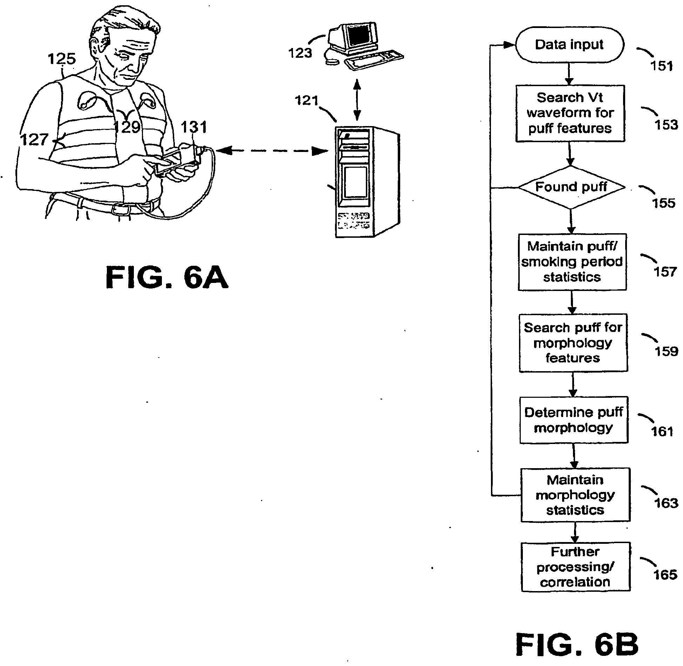

In order to perform normal daily waking and sleeping activities, a monitored subject should be only minimally, if at all, constrained by a monitoring device. In preferred embodiments, therefore, physiological sensors are attached to, or affixed to, or carried by, or incorporated in or as part of ordinary wearable items that are unobtrusive, comfortable, and useable without assistance. Suitable wearable items include garments, jackets, bands, patches, and the like, made from a variety of materials, particularly elastic materials to insure a snug fit; they can be donned in one piece or include zippers, Velcro, snaps, and the like, that are joined after donning. Sensors can be incorporated into garments in many ways, for example, by weaving, or knitting, or braiding into a garment's fabric; or by being sewn or carried on, or mounted in, or attached to the garment; also flexible sensors can be glued, printed, sprayed and so forth onto inner or outer garment surfaces. See, e.g., U.S. patents 6,551,252 and 6,047,203. For example, Fig. 6A illustrates shirt-like garment 125 to which sensors 127 and 129 are affixed or attached. This garment can be joined by a zipper, Velcro, or the like along the ventral midline; optionally it can be one piece.

Many types of sensors can be incorporated in wearable, monitoring items. One useful physiological sensor, referred to herein generically as a "size sensor", gathers signals responsive to size indicia describing portions of a monitored subject's body, e.g., the torso, the neck, the extremities, or parts thereof. Size sensor signals can be processed to yield information about organ system functioning. In particular, signals from size sensors at one or more levels of the torso, e.g., at an abdominal level and at a rib cage level, can be interpreted using a two-component breathing model in order to determine respiratory rates, respiratory volumes, respiratory events, and the like. See, e.g., U.S. patents 6,551,252; 5,159,935; 4,777,962; and U.S. patent application 10/822,260.

Preferred size sensors are based on inductive plethysmographic (IP) technologies. Briefly, the impedance of a conductive element is known to reflect size and shape of the element. Therefore, the impedance of a conductive element configured to lie on a portion of a body part, or to partially or fully encircle a body part, or otherwise arranged on the body of a subject changes as the size of the underlying body part changes due to, e.g., respirations, pulsations, voluntary motions, cardiac activity, and the like. IP technology measures this impedance and consequently reflects such physiological functioning. However, useful size sensors can be based on diverse other technologies known in the art. Active elements of size sensors can be based on thread and fabric technologies. The exemplary garment of Fig. 6A includes two size sensors 127 at rib cage and abdominal levels, which return signals that can be processed into respiratory information.

In addition to size sensors providing respiratory and/or cardiac information, wearable items can include diverse additional sensors for other physiological and/or non-physiological parameters of a monitored subject. For example, accelerometers can sense current activity level and body posture or orientations; thermistors can sense skin or body core temperature; pulse oximeters can sense blood oxygen level. Further, electrodes in electrical communication with the subject can sensor such electrical activities as electrocardiogram ("ECG") signals, electroencephalogram ("EEG") signals, electro-oculogram ("EOG") signals, electro-myogram ("EMG") signals (of the orbital, facial and other muscles), skin conductance or resistance, and the like. These electrodes can be as known in the art of can be fabric based, or are otherwise flexible. Additional sensor can be mounted or incorporated in various manners. Fig. 6A illustrates ECG electrodes 129 in contact with the subject through cutouts in garment 125. Alternatively ECG electrodes can be mounted on the inner surface of garment 125 to be directly in contact with the subject's skin, thus obviating garment cutouts.

Wearable items are usually accompanied by local electronic units, referred to herein as portable data units ("PDUs") that are sufficiently compact and lightweight to be carried on or by the monitored subject. PDUs preferably include IP sensor electronics and also electronics for operating other sensors, and for retrieving and digitizing (if necessary) sensor data. See, e.g., U.S. patent 6,551,252. PDUs are also preferably in two-way communication, wired or wireless with physically local or physically remote external server systems so that sensor data can transmitted in real time. Alternatively, sensor data can be stored for later access on, e.g., flash memory, or magnetic media, hard drives, or the like.

External server systems typically receive and store data from one or more PDUs, perform additional processing of received data to determine further physiological information, and format and display received for monitoring personnel. In preferred embodiments, the methods of this invention are implemented on such external systems. Figs. 6A illustrates PDUs 131 connected by wire to the garment sensors and in wireless contact with external server system 121. Server system 121 can be a PC-type or workstation-type computer both of which are well known in the art. User interface devices, such a display, mouse, and keyboard 123, preferably accompany server systems for use by monitoring personnel.

Sensor signal processing whether in a PDU or in an external systems generally includes filtering, digitization, noise limiting, extraction of relevant signal components,, specific processing of respiratory size sensor signals. See, e.g., U.S. patent 6,413,225; 5,159,935; 4,834,766; 4,777,962; and U.S. application 10/822,260. Signals from other sensors can also be processed: R-waves can be recognized in ECG signals using known methods; accelerometer signals can be low and high pass filters to extract posture information and activity level information, respectively; and so forth. See, e.g., U.S. application 10/991,877.

This methods of this invention are performed on software or firmware programmable systems. In the case of software programming, methods are coded in standard computer languages, such as C, C++, or in high level application languages, such as Matlab and associated toolboxes (Math Works, Natick, MA). Code is then translated or compiled into executable computer instructions for controlling a microprocessor or similar. In the case of firmware programming, higher level method specifications written in software languages or hardware languages such as VHDL, are generally translated into bit codes by tools supplied by the manufacturer of the hardware part that is being programmed. For example, manufacturer's tools prepare bit-streams for configuring FPGAs. The invention also includes software distributions, e.g., on computer-readable media, having encoded the method of this invention for execution on a computer or for programming a firmware device.

### EXEMPLARY EMBODIMENTS OF THE SMOKING ANALYSIS METHODS

The smoking analysis methods of this invention are implemented as software programs that are preferably executed on server systems that receive data from one or more ambulatory monitoring devices. For example with reference to Fig. 6A, these software programs can be executed on server system 121 with input data received from monitoring garment 125 through PDU 131.

Fig. 6B illustrates a flowchart for an exemplary software implementation. The first : steps searches for puffs in input data 151 received directly or indirectly from an ambulatory monitoring device. A tidal volume (Vt) waveform is obtain for this data and searched 153 for breaths with the above-described puff-indicating features. If a breath is found having sufficient puff-like features, it is determined that a puff has been found 155; otherwise the program continues searching Vt data. Sufficient features can be found if a breath has a threshold number of features that are optionally weighted by their significance, or if a breath has a number of required features and a threshold number of optional features, or the like. Optionally, puff identification can be suspended during periods of activity, as indicated by input accelerometer data, sufficient to cause puff identification to be unreliable. Puffs and their times of occurrence are then summarized 157 by known statistical methods (e.g., totals, means, standard deviations, and the like) to produce characteristics describing a subject's overall smoking behavior.

Having identified a puff, the next steps determine its morphology. Accordingly, the identified puff is searched for key morphological features 159 such as type and volume of inhalation, type and volume of exhalation, duration of breath holding, if any, and the volume during breath holding, and the like. Puff morphology is determined from the features found and statistical data is maintained 163 on the types of subject puffs, such as average duration and its standard deviation, percent of breath holding time, relative tidal volume, and the like. Number of puffs of various types, such as the above-described types, can also be maintained. The implementation then resumes receiving input data 151 and searching it 153 for puffs.

Once sufficient data is accumulated, it is preferably further processed. At least, it is displayed for analysis. Further, it can be correlated with other physiological monitoring data. Also, with reference to smoking behavior databases, prognostic information can be estimated for the subject. Also, it can be used as biofeedback to aid in smoking cessation or amelioration.

A number of references are cited herein, including patents and patent applications, the entire disclosures of which are incorporated herein, in their entirety, by reference for all purposes. Further, none of these references, regardless of how characterized above, is admitted as prior to the invention of the subject matter claimed herein.

Although specific features of the invention are shown in some drawings and not in others, this is for convenience only as each feature may be combined with any or all of the other features in accordance with the invention. The words "including", "comprising", "having", and "with" as used herein are to be interpreted broadly and comprehensively and are not limited to any physical interconnection. Moreover, any embodiments disclosed in the subject application are not to be taken as the only possible embodiments. Other embodiments will occur to those skilled in the art and are within the following claims.

## Claims

1. A method for characterizing a subject's smoking behaviour comprising:
receiving (151) ambulatory monitoring data, including data reflecting tidal volume (Vt);
searching (153) received Vt data for candidate puffs which are breaths having one or more features signaling a puff, wherein:
one feature signaling a puff is a breath with a duration and amplitude both of which are proximately 1.5 to 2.0 times longer or greater, respectively, than adjacent breaths; and
a further feature signaling a puff is a breath having a substantially single inspiratory phase, which is interrupted by a period at a substantially constant lung volume, and a substantially single expiratory phase;
determining (155) whether or not a candidate puff is an actual puff based on the number and type of all puff-signaling features recognized; and
storing puff occurrence data, including times of occurrence data, and puff characteristic data.

2. The method of claim 1 wherein the one or more features signaling a puff further include one or more of inspiratory durations, and/or flow rates and/or volumes; or expiratory durations, and/or flow rates and/or volumes; or duration of the period between an expiration and the following inspiration.

3. The method of claim 1 wherein actual puff determination is suspended during periods of activity sufficient to cause puff determination to be unreliable.

4. The method of claim 1 further comprising characterizing the subject's overall smoking behavior in dependence on the stored puff occurrence data.

5. The method of claim 1 further comprising searching (159) at least one actual puff for morphological features; storing puff morphology data; and characterizing the subject's puff and manner of smoking in dependence on the stored puff morphology data.

6. The method of claim 5 wherein the puff morphology features include one or more of inspiratory durations, and/or flow rates and/or volumes; or expiratory durations, and/or flow rates and/or volumes; or duration of the period between an expiration and the follow inspiration.

7. The method of claim 5 further comprising correlating the subject's overall smoking behavior and the subject's puff behavior with indicia of the functioning of other physiological systems.

8. The method of claim 5 further comprising providing biofeedback to the subject in dependence at least in part on the stored puff occurrence data and on the stored puff morphology data.

9. A system for characterizing a subject's smoking behavior comprising:
an ambulatory monitoring device (131) providing physiological monitoring data, including data reflecting Vt; and
a computer (121) operatively coupled to the ambulatory monitoring device (131) and including a computer-readable memory with encoded instructions for performing the methods of claim 1.

10. The system of claim 9 wherein the encoded instructions for further performing the methods of claim 5.

11. The system of claim 9 wherein the computer is operatively coupled to a plurality of ambulatory monitoring devices (131).

## Patentansprüche

1. Verfahren zum Charakterisieren des Rauchverhaltens einer Person, aufweisend:
Empfangen (151) ambulanter Beobachtungsdaten, beinhaltend Daten, welche das Atemvolumen (Vt) widerspiegeln;
Durchsuchen (153) der empfangenen Vt Daten nach Kandidatenzügen, welche Atemzüge sind, welche eine oder mehrere Merkmale aufweisen, welche einen Zug signalisieren, wobei:
ein Merkmal, welches einen Zug signalisiert, ein Atemzug mit einer Länge und Amplitude ist, welche beide annähernd 1,5- bis 2,o-mal länger bzw. größer sind als benachbarte Atemzüge; und
ein weiteres Merkmal, welches einen Zug signalisiert, ein Atemzug ist, welcher eine im wesentlichen einzige Inspirationsphase aufweist, welche durch eine Periode bei einem im wesentlichen konstanten Lungenvolumen unterbrochen ist, und eine im wesentlichen einzige Exspirationsphase;
Ermitteln (155), ob ein Kandidatenzug ein tatsächlicher Zug ist oder nicht, basierend auf der Anzahl und dem Typ aller erkannten Zugsignalisierenden Merkmale; und
Speichern von Zugvorkommen-Daten, beinhaltend Daten betreffend die Zeiten des Vorkommens, und Zug-charakteristische Daten.

2. Verfahren nach Anspruch 1, wobei die einen oder mehreren Merkmale, welche einen Zug signalisieren eines oder mehrere beinhalten von einer Inspirationsdauer, und/oder Flussrate und/oder Volumen; oder Exspirationsdauer, und/oder Flussraten und/oder Volumen; oder Dauer der Periode zwischen einer Exspiration und der nachfolgenden Inspiration.

3. Verfahren nach Anspruch 1, wobei die tatsächliche Zugermittlung ausgesetzt wird während Perioden von Aktivität, welche ausreichend ist, zu veranlassen, dass die Zugermittlung unzuverlässig ist.

4. Verfahren nach Anspruch 1, weiter aufweisend das Charakterisieren des Gesamtrauchverhaltens der Person in Abhängigkeit von den gespeicherten Zugvorkommen-Daten.

5. Verfahren nach Anspruch 1, weiter aufweisend das Durchsuchen (159) zumindest eines tatsächlichen Zugs nach morphologischen Merkmalen; Speichern von Zug-Morphologiedaten; und
Charakterisieren des Zugs und der Art des Rauchens der Person in Abhängigkeit von den gespeicherten Zug-Morphologiedaten.

6. Verfahren nach Anspruch 5, wobei die Zug-Morphologiemerkmale eines oder mehrere beinhalten von Inspirationsdauer, und/oder Flussraten und/oder Volumen; oder Exspirationsdauer, und/oder Flussraten und/oder Volumen; oder Dauer der Periode zwischen einer Exspiration und der nachfolgenden Inspiration.

7. Verfahren nach Anspruch 5, weiter aufweisend das Korrelieren des Gesamtrauchverhaltens der Person und des Zugverhaltens der Person mit Indizien der Arbeitsweise anderer physiologischer Systeme.

8. Verfahren nach Anspruch 5, weiter aufweisend das Bereitstellen von Biofeedback an die Person in Abhängigkeit zumindest teilweise von den gespeicherten Zug-Vorkommendaten und der gespeicherten Zug-Morphologiedaten.

9. System zum Charakterisieren eines Rauchverhaltens einer Person, aufweisend:
ein ambulantes Beobachtungsgerät (131), welches physiologische Beobachtungsdaten bereitstellt, beinhaltend Daten, welche Vt widerspiegeln; und
ein Computer (121), welcher funktional mit dem ambulanten Beobachtungsgerät (131) verbunden ist und einen computer-lesbaren Speicher mit kodierten Anweisungen beinhaltet, um die Verfahren nach Anspruch 1 durchzuführen.

10. System nach Anspruch 9, wobei die kodierten Anweisungen, weiter zum Durchführen eines Verfahrens nach Anspruch 5 sind.

11. System nach Anspruch 9, wobei der Computer funktional mit einer Vielzahl von ambulanten Beobachtungsgeräten (131) verbunden ist.

## Revendications

1. Un procédé de caractérisation du comportement de fumeur d'un patient, comprenant :
la réception (151) de données de suivi ambulatoire, comprenant des données reflétant le volume respiratoire courant (Vt) ;
la recherche (153) de données Vt reçues pour des bouffées supposées qui sont des respirations présentant une ou plusieurs caractéristiques révélant une bouffée, où :
l'une des caractéristiques révélant une bouffée est une respiration présentant une durée et une amplitude qui sont toutes deux approximativement 1,5 à 2,0 fois plus longues ou plus grandes, respectivement, que les respirations adjacentes ; et
une autre caractéristique révélant une bouffée est une respiration présentant une phase inspiratoire substantiellement unique, qui est interrompue par une période à volume pulmonaire substantiellement constant, et une phase expiratoire substantiellement unique ;
la détermination (155) si une bouffée supposée est ou non une bouffée effective sur la base du nombre et du type de toutes les caractéristiques révélant une bouffée ; et
la mémorisation de données de survenue de bouffée, comprenant des instants de survenue de bouffée et des données de caractéristiques de bouffée.

2. Le procédé de la revendication 1, dans lequel les une ou plusieurs caractéristiques révélant une bouffée comprennent en outre une ou plusieurs parmi : des durées et/ou des débits et/ou des volumes inspiratoires ; ou des durées et/ou des débits et/ou des volumes expiratoires ; ou la durée de la période entre une expiration et l'inspiration suivante.

3. Le procédé de la revendication 1, dans lequel la détermination d'une bouffée effective est suspendue pendant des périodes d'activité suffisantes pour que la détermination d'une bouffée ne soit pas fiable.

4. Le procédé de la revendication 1, comprenant en outre la caractérisation du comportement global de fumeur du patient en fonction des données de survenue de bouffée mémorisées.

5. Le procédé de la revendication 1, comprenant en outre : la recherche (159) de caractéristiques morphologiques d'au moins une bouffée effective ; la mémorisation de données de morphologie de bouffée ; et la caractérisation de la bouffée du patient et de la manière de fumer en fonction des données morphologiques de bouffée mémorisées.

6. Le procédé de la revendication 5, dans lequel les caractéristiques de morphologie de bouffée comprennent une ou plusieurs parmi : des durées et/ou des débits et/ou des volumes inspiratoires ; ou des durées et/ou des débits et/ou des volumes expiratoires ; ou la durée de la période séparant une expiration et l'inspiration suivante.

7. Le procédé de la revendication 5, comprenant en outre la corrélation du comportement global de fumeur du patient et du comportement à la bouffée du patient avec des indices du fonctionnement d'autres systèmes physiologiques.

8. Le procédé de la revendication 5, comprenant en outre la délivrance d'une biorétroaction au patient au moins en partie en fonction des données de survenue de bouffée mémorisées et des données de morphologie de bouffée mémorisées.

9. Un système pour la caractérisation du comportement de fumeur d'un patient, comprenant :
un dispositif de suivi ambulatoire (131) délivrant des données de suivi physiologique, comprenant des données reflétant le Vt ; et
un calculateur (121) couplé de manière opérante au dispositif de suivi ambulatoire (131) et comprenant une mémoire lisible par calculateur avec des instructions codées pour mettre en oeuvre les procédés de la revendication 1.

10. le système de la revendication 9, dans lequel les instructions sont codées pour en outre mettre en oeuvre les procédés de la revendication 5.

11. Le système de la revendication 9, dans lequel le calculateur est couplé de manière opérante à une pluralité de dispositifs de suivi ambulatoire (131).
